(19) Europäisches Patentamt
European Patent Office
Office européen des brevets

(11) **EP 2 537 548 B1**

(12) **EUROPEAN PATENT SPECIFICATION**

(45) Date of publication and mention
of the grant of the patent:
**03.02.2016 Bulletin 2016/05**

(51) Int Cl.:
*A61M 11/00* *(2006.01)*       *A61M 11/06* *(2006.01)*
*A61M 15/00* *(2006.01)*

(21) Application number: **12172518.8**

(22) Date of filing: **19.06.2012**

(54) **A device for controlling and monitoring of an apparatus for generating an aerosol during administration of a dose of a substance by way of inhalation**

Vorrichtung zur Steuerung und Überwachung eines Aerosolerzeugers bei der Substanzabgabe durch Inhalation

Appareil pour contrôler et surveiller un générateur d'aérosol pendant l'administration d'une dose de substance par voie d'inhalation

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **22.06.2011 PL 39540111**

(43) Date of publication of application:
**26.12.2012 Bulletin 2012/52**

(73) Proprietor: **Secura-Nova Spolka z Ograniczona Opdowiedzialnoscia**
**00391 Warszawa (PL)**

(72) Inventors:
• **Florkiewicz, Emil**
**98-200 Sieradz (PL)**

• **Florkiewicz, Maciej**
**98-200 Sieradz (PL)**
• **Pirozynski, Michal**
**00-358 Warszawa (PL)**

(74) Representative: **Balinska, Ewa**
**Polservice**
**Kancelaria Rzecznikow**
**Patentowych sp. z o.o.**
**Bluszczanska 73**
**PL-00-712 Warszawa (PL)**

(56) References cited:
WO-A1-00/01434        WO-A1-2006/013952
WO-A2-92/15353        WO-A2-97/48431
US-A1- 2003 205 229    US-A1- 2006 201 499

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

An object of the invention is a device for controlling and monitoring of a device for generation of an aerosol during administering of a medicament dose by inhalation method, and especially for controlling different types of devices for inhalation and for monitoring of a medicament dose administered by them.

A therapeutic method through introduction of a medicament in an aerosol form directly into respiratory tract, i. e. by inhalation, is known, as well as many devices are known for generation of an aerosol and administering of a medicament substance dose in an aerosol form by inhalation method, which generally are called as inhalers or nebulisers, in the case of devices using compressed air to disperse a medicament dose.

In order to obtain a therapeutic effect, in the prior art inhalers, a medicament dose concentration is set in a very wide limits of tolerance (up to 200%) based on tabular data. The volume of a medicament for use in a therapy is selected in a wide range of variations from 100% to 25% of a volume comprised in a factory packaging. An amount of an optional excipient (diluent) mixed with the medicament in order to obtain a larger volume of a solution for nebulisation is selected in a wide range of variations (from 0% to 400%) in relation to the volume of the medicament used.

These parameters are always set and used, regardless of the manner of their acquisition, processing and eventual fixation. In the prior art, for example according to US patent 6,606,989, on the basis of known values of: a medicament concentration, its volume allocated to a therapy and a volume of solvent used, it is possible to precisely define medicament dose in any volume of a mixture intended for nebulisation.

The disadvantage of this approach lies in the fact that a control of a medicament dose intended to achieve a therapeutic effect is only possible up to start of a nebulisation. Since the start of nebulisation control of a dose intended to achieve a therapeutic effect is not possible.

Inhalation can be conducted using a freely chosen, well-known, inhalation device, for example, a nebuliser. Such devices are characterized by large range of parameters which are key to their work but independent. Differences in consumption of a liquid phase (medicament solution) volume per unit of time come up to 300% depending on a nebuliser type. Differences in volume of air used by them to achieve aerosol per unit of time come up to 300% depending on a compressor used.

The respirable fraction obtained from these devices varies from 50% to 98%.

The key problem to understand essence of phenomena is that conducting of an inhalation means in fact simultaneous operating of a group of freely selected parameters: an expected dose in a range of values from 100% to 200% in relationship to the lowest dose used, including a medicament poured into a nebuliser in a range of values from 25% to 100% of a volume comprised in a factory packaging, including an optional diluent in a range of values from 0% to 400% in relation to used volume of a medicament, taking into account the a nebuliser output in a range of values from 100% to 300% in relation to the lowest output of the typical nebuliser, including an air flow rate in a range of values from 100% to 300% in relation to the lowest flow rates used in nebulisers. The probability of reaching a bronchial by an aerosol composed of carefully tuned sets (respirable fraction) ranges from 50%-98%. The probability of reaching a bronchial by an aerosol composed of accidentally tuned sets (respirable fraction) is not possible to be determined. In the prior art devices, the above-mentioned parameters are not taken into account, or they are taken into account only in a very limited range. Despite the above reservations, in the previously accepted practice when changing used medicament inhalation device any parameters characteristic for a medicament are not changed, which leads to significant inaccuracies in generation and metering of recommended dose.

There are many types of devices for continuous operation in which an air from a compressed air source is fed to a nebulizer through a pressure hose After introduction into a nebuliser specific volume of a liquid designed for inhalation, prepared in accordance with a known method, a device is started that produces an aerosol until all produced liquid is finished, regardless of a patient behaviour, i.e. without taking into account relationship between generation of an aerosol and bringing of the a reference dose to a patient's respiratory tract during nebulisation.

The disadvantage of such devices is that there is no possibility of introducing or using of data describing a composition and volume of dosed solution, resulting in a further disadvantage of this approach which lies in the fact that during treatment differences in a density of the an aerosol resulting from changing and different for each device ratios of a liquid flow rate to an air volume are not taken into account. In effect, this means no control over a medicament dose contained in a volume of an aerosol.

Another disadvantage is inability to make and use during operation of a device of data on consumption of a liquid phase volume and an air volume, which makes impossible assessment of the patient's ability to use the whole volume of an aerosol. Especially in the case of small children there is a situation in which a volume of an aerosol generated by the device is higher than a volume of respiratory track of a child, which causes losses of aerosol during inspiration. In the previously known devices it is not possible to estimate those losses. Similarly, the disadvantage of known devices is the lack of possibility to use for a treatment of a volume of an aerosol produced during exhalation phase of a patient, which causes global losses from 50% to 80% of aerosol, and thus not only irretrievable loss of medicament dose planned to achieve a therapeutic effect but also additional environment pollution.

It is known, for example, an inhaler that contains a source of compressed air flowing through a pressure hose to an air supply control valve. This valve is connected to a nebuliser through the further pressure air line, which terminates in a mouthpiece, to which an air hose is also connected which is at the other end connected to the a pressure sensor as in the embodiments disclosed in US patent No 6,606,989 B1 and DE 19939417A1. After a specific volume of a liquid designed for inhalation, prepared in a known manner, is entered into a nebuliser, an aerosol is produced cyclically, only during certain periods, depending on the method used for controlling of administering of an aerosol.

In the solution proposed in US Patent 6,606,989 an inspiration time of a particular patient is determined before starting of a nebulisation and on this basis a device for nebulisation is properly adjusted in order to avoid both, unnecessary and too short spraying of a medicament. A patient during inhalation receives a signal from the device when he/she should start and stop inspiration. By means of the device disclosed in this patent, however, interruptions of inspiration, breaks in rest condition, etc. In a patient undergoing inhalation cannot be taken into account. Once started process of aerosol spraying takes so long and continues with such intensity, as it was previously set without possibility of correction and real control of administered medicament dose.

According to the solution disclosed in DE 19939417 A1 the proposed device for inhalation ensures synchronization of aerosol administering with an inspiration, i.e. with a breath rhythm of a patient, through recognizing of an inspiration phase and running of an aerosol production at this stage by supplying compressed air to a nebuliser through a valve. A control system of the device detects with the pressure sensor inspiration phase and exhalation phase, In which phase the valve closes air supply to the nebuliser. However, in that device, the problem of medicament dose calculations as a function of the aerosol density, and thus the problem of the medicament dosage, was not solved. According to the embodiment of DE 19939417 A1 and the method claimed in it, a volume of an aerosol in individual portion, or the a volume of an aerosol summed from all inspirations, or - which is related to the mentioned disadvantages - a medicament dose contained in a volume of generated aerosol, cannot be determined. The disadvantage of the method is also that the problem of stopping of inhalation after reaching a pre-set dose is not resolved.

Another drug delivery apparatus is known from the US 2003/0205229 A1, comprising drug delivery apparatus, a sensor for monitoring a breathing pattern of a patient and a controller for control drug delivery device to deliver drug dose in pulses starting from begin of inhalation by the patient monitored by the sensor.

There are also known inhalers and inhalation apparatuses that are provided with controlling means that control delivery of the determined medicament dose to the patient's respiratory airways using the patient's inhalation pattern. Such inhalers are disclosed for example in US 2006/0201499 A1, WO 92/15353, WO 97/48431, WO 2006/013952 A1 and WO 00/01434

Solutions known from the state of the art do not take into account the situation in which configuration of a compressor and a nebuliser is changed, and they do not provide methods or mechanisms to solve the related problems of dispensing appropriate medicament dose, taking into account that an aerosol after change of the configuration has completely different set of parameters.

Solutions known from the state of the art do not provide also for the archiving of data showing the actual patient's cooperation with a device, which means that this cooperation cannot be reproduced and analysed.

The invention provides a solution of the above mentioned problems associated with the exact determination of the medicament dose, which in fact has been brought into the respiratory tract of the patient taking into account all the essential parameters of the medicament, aerosol parameters, and set of devices applied to administer of the medicament dose, also when changing the configuration, i.e. change referred to the one or more devices in the system or change of the administered medicament. The invention solves the problem of association of a capacity of a device used for continuous generation of an aerosol generation with efficiency of supplying of a pre-set dose to a patient's respiratory tract during nebulisation at any change associated with parameters of both a device used and parameters of administered medicament and any change in their combination.

The above problem is solved according to the present invention by a device for controlling and monitoring of a supervised device for generation of an aerosol during metering of a medicament dose, in particular for supervision of a process of generation of an aerosol during metering of administered medicament dose defined as a pre-set dose, and especially of a supervised inhalation device employed for continuous generation of an aerosol comprising a mouthpiece, which device is configured to cooperate with said supervised device and can be connected to said supervised device that is in the form of:

- an inhaler for generation of an aerosol by pressure method comprising at least a power cord, a nebulizer, a compressor and,
when it is of the type without an electronic control of an aerosol production further having pressure lines P1,P2, or
when it is of the type having its own control system for controlling generation of an aerosol further having a power cord; or
- a ultrasonic inhaler for generation of an aerosol by ultrasonic method having a ultrasonic probe and a power supply cord of said probe,

and the device for controlling and monitoring comprising:

a power supply module, a pressure sensor with a port, a signal module, a data analysis module including a FLASH memory in it, a data transmission and acquisition module with a connection unit, preferably USB port, a time standard and time synchronization module transmitting signals to said data analysis module, that are time standard signals, during proceeding the inhalation in time and determining single measurement cycle and a control module for controlling said supervised device, that module is further provided with:

- connection sockets for connection to main supply line that is said power cord and/or said power cord of the probe
- at least one valve, preferably electric valve with an output port and an input port, at said valve there is connection between the input port and the output port, wherein pressure lines can be connected to the device so that, the stream of compressed air from compressor is directed by said pressure line P1 to the input port in the control module and the output port of this module is connected by said the other pressure line P2 to the nebulizer, wherein in use, during metering medicament dose, the device together with said supervised inhalation device are connected at this stage of operation in one system, the further operation of which is dependent on individual patient breathing pattern and which is controlled by a patient breath, and further wherein the device is configured:
- to perform initial calibration and adjustment step of the device to operation with said specific supervised inhalation device, to which it is attached, during which step values describing a selected method and a selected inhalation device connected to the device which is supervised are loaded via said USB port and said data transmission and acquisition module through recording into the FLASH memory of said data analysis module of an aerosol volume, in which a dose equal to the pre-set dose in litres is contained, a time required to generate an aerosol volume containing the pre-set dose in minutes, and threshold pressure value for said pressure sensor, and
- to perform an individual calibration for each said system formed with determining its:
- individual threshold pressure value for the pressure sensor below which the device recognizes an inspiration phase, which value is interpreted as beginning of inspiration phase and exhalation phase; and
- threshold aerosol volume value, as well as,
- threshold time value of duration of aerosol generation,
- to control operation of said supervised device, and wherein during metering said dose a pressure drop below the threshold pressure value is detected, which means start of inspiration phase and start of generation aerosol by said supervised inhalation device, which generation is continued until the moment of reaching again of the threshold pressure value or higher value, that is interpreted as beginning of exhalation phase, and wherein a process of generation of an aerosol during metering of a dose in supervised device is completed at the time when a volume of aerosol used for metering of a dose, which is a sum of instantaneous volumes measured in single measurement cycles, exceeds the threshold value of an aerosol volume described as such a volume of an aerosol, in which a dose equal to the pre-set dose is contained and/or when a time used effectively for generation of an aerosol during metering of the dose, which is a sum of instantaneous times of generation of an aerosol, measured in single measurement cycles, exceeds the threshold time value of generation of said aerosol, determined by total time required to disperse the pre-set dose.

In one aspect of the invention the power supply module of the controlling and monitoring device for power supplying is connected to the pressure sensor, as well as to the data analysis module and to the time standard and time synchronization module, while its other connections are connected to connections of the control module for controlling the supervised device, respectively, while the pressure sensor, transmitting a signal reflecting a current pressure at the signal cable, connecting supervised inhaler device and the pressure sensor by the port is connected to the data analysis module, to which the time standard and time synchronization module is also connected, which the data analysis module sends control signals, via and adequate connection, to the control module for controlling said supervised device, wherein the data analysis module, in order to send and receive signals is also connected to the data transmission and acquisition module and the signal module, respectively, to which the data transmission and acquisition module has also separate connection in order to transmit signal on a status of using of the connection unit, preferably in the form of USB port.

In another aspect of the invention in the device for controlling and monitoring the signal module comprises signal diodes, preferably LEDs, of which one LED indicates a power supply connection, another LED indicates a connection to the communication and data transmission module, while the further another LED indicates an inspiration and metering phase, and the other LED indicates stopping of metering.

According to further another aspect of the invention the device for controlling and monitoring is connected to said supervised device in such a way that

- the port of the pressure sensor of the controlling and monitoring device is connected to the signal cable connecting by the port said supervised inhaler device to the pressure sensor of the controlling and monitoring device and outgoing from the nebulizer or from a mouthpiece of the supervised device; and

- the power cord of said supervised device is connected to a corresponding connection of the control module for controlling operation of the supervised device, namely: a socket, and/or
- the power cord of an ultrasonic probe is connected to the other socket of the control module for controlling operation of supervised device; and/or
- the one pressure line P1 outgoing from the compressor of said supervised device is connected to the input pressure port of the control module, while the pressure output port of the control module is connected by the other pressure line P2 to the nebuliser of said supervised device.

In the device according to the invention for controlling and monitoring of the device for generation of the aerosol during metering of a medicament dose by inhalation method during generation of an aerosol by the supervised device, exceeding of a pressure threshold value is read in the pressure line, connected to a mouthpiece of the supervised device, preferably an inhaler, which means the start of the inspiration phase and the start of a generation of an aerosol by the supervised inhalation device, until the moment of crossing again the threshold pressure value in the pressure line, which is interpreted as an exhalation phase, or a break of breathing, wherein generation of an aerosol and metering of the dose by the supervised inhalation device is then cut off and the generation of aerosol and metering of the dose by the supervised inhalation device is restarted after the pressure sensor detects crossing again of the threshold pressure value in the pressure line, interpreted as a re-start of the inspiration phase, wherein these steps are repeated until the completion of the metering. The supervised device is combined into a single unit with a controlling and monitoring device for aerosol generation during metering of the dose, which unit is controlled according to the inspiration and exhalation respiratory phases, wherein individual calibration of each unit formed in this way is performed, determining its individual threshold pressure value for the pressure sensor, which is interpreted as the beginning of the inspiration phase and exhalation phase and the threshold aerosol volume value and the threshold time value of the duration of the aerosol generation. Then the metering of the pre-set dose is started, during which, the individual measured instantaneous volumes of the generated aerosol, used to metering of the dose in each single test cycle are recorded and summed by the controlling and monitoring device at a real time, as well as the instantaneous aerosol generation times measured in single measurement cycles are recorded and summed, and the metering of the supplied dose is controlled and monitored. The process of the generation of the aerosol during metering of the dose in supervised device is completed at a time when the volume of the aerosol used for metering of the dose, which is a sum of the instantaneous volumes measured in single measurement cycles, exceeds the threshold value of the aerosol volume described by the volume of the aerosol, in which the dose equal to the pre-set dose is contained and/or when the time used effectively for generation of the aerosol during metering of the dose, which is the sum of the instantaneous times of the generation of the aerosol, measured in single measurement cycles, exceeds the threshold time value of the generation of the aerosol, determined by the total time required to disperse the pre-set dose.

In a preferred embodiment, generation of an aerosol during metering of the dose by the supervised device ends at the moment of exceeding the pre-set value of the threshold aerosol volume and/or of the duration time of the generation of aerosol at a time when at least one of the two values exceeds the relevant threshold value.

In one embodiment of the invention a signal from the pressure sensor having value equal to or greater than the determined threshold pressure value is detected by the data analysis module, which is identified by the controlling and monitoring device as exhalation phase and in this phase neither the aerosol volume as the volume used, nor the time as the time of the aerosol generation used to effectively metering of the pre-set dose is counted up to the moment in which the pressure sensor signal value reaches again the threshold pressure value or lower than it, what will be read as an inspiration phase, during which aerosol generation will re-start during metering of the dose, wherein after restarting of the metering of the dose the instantaneous values of the aerosol volume and the duration time of the aerosol generation are again summed and counted and the counted values are compared by the data analysis module to the total threshold values of the aerosol volume and the time duration of the aerosol generation, defined in an auto-calibration step of the controlling and monitoring device.

According to yet another aspect of the invention, preferably during inspiration, in any individual measurement cycle, sampling and processing of the pressure sensor signal by the data analysis module and responding to its changes is stopped at the moment of recognition that the summed value of the aerosol volume used to metering is equal or greater than the threshold volume, described as the aerosol volume, In which a dose equal to the reference dose is contained, or at the moment of recognition that the summed value of the time taken to generation of the aerosol during metering is equal or greater than the threshold time determined by the total time required to disperse the pre-set dose, and additionally the signals are also sent to the external data communication and acquisition module about finishing of the communication via the USB port and recording data to a data storage device, preferably on a micro SD card, to the module for controlling of the operation of the supervised device, preventing further generation of the aerosol and to the signal module about displaying information on completion of aerosol supplying and dose metering.

The device of the invention allows precise control of operation of any set of inhalation devices, even when changing their configuration, for example, when changing the compressor and/or nebuliser used, and also the type of the medicament used and ensures administering of the desired, pre-set dose of a substance, regardless of the set of equipment and type of substance used and the characteristics of the patient's breath.

The device of the present invention resolve the problem of providing possibility to monitor in real time, with known accuracy, the phenomena occurring in any operating device for nebulisation, as well as set of devices for spraying medicament or medicated solution prepared in a known manner, taking into account the consequences resulting from any combination of the expected or pre-set dose, the dose used for this method, the volume of the solvent used, the nebuliser output and the air volume delivered by the compressor.

The device of the present invention provides full versatility, i.e. it allows cooperation of the device according to the present invention with any inhalation device of any kind, to the use of the therapeutic method by the inhalation method and it can be connected in a wide range of variants and configurations. The phenomena monitored with the known accuracy can be archived on an ongoing basis, which allows their subsequent analysis.

The device of the invention allows for carrying out of a simulation of a nebulisation treatment taking into account the consequences resulting from any combination of the expected dose planned to practical using, the dose applied to the method, the volume of the solvent used, the nebuliser output and the air volume delivered by the compressor. It also allows for performing an analysis of the patient's ability to use other therapeutic methods than nebulisation, it preferably allows to optimize selection of dry powder inhalers. By using the device of the invention it is possible to reproduce in laboratory conditions the situations taking place in the past

The embodiments of the object of the invention are shown in the drawings, in which

fig. 1 shows a schematic diagram of the device of the present invention in a first embodiment,
fig. 2 shows a schematic diagram of the device of the present invention in a second embodiment,
fig. 3A - shows a schematic diagram of a method for connecting the device of the invention to a supervised device,
fig. 3B - shows a schematic diagram of another method for connecting the device of the invention to a supervised device,
fig. 4 - shows a schematic diagram of a further method for connecting the device of the invention to a supervised device, and
fig. 5 - shows a schematic diagram of a further method for connecting the device of the invention to another type of a supervised device.

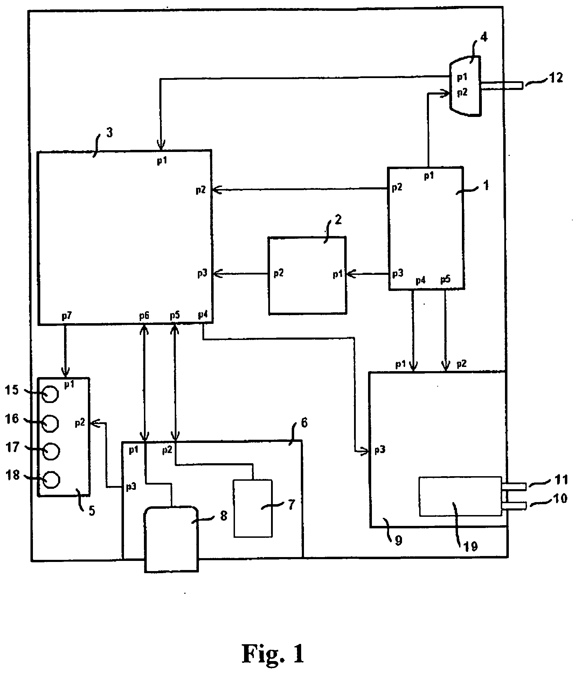

In a first embodiment of the invention shown in fig. 1, a controlling and monitoring device U of a supervised device A for generation of an aerosol used in order to supervise a process of an aerosol generation during inhalation and metering of a dose administered by the device, comprises the power supply module 1, the time standard and time synchronization module 2, the data analysis module 3 comprising a FLASH memory in it, the pressure sensor 4, the signal module 5, the data transmission and acquisition module 6 and the control module 9 for controlling the supervised device, which can comprise at least one valve 19, preferably the electric valve 19.

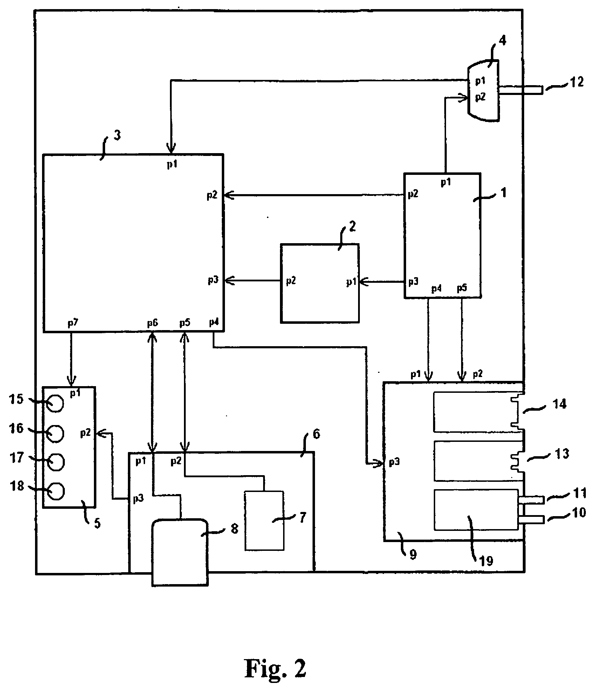

An alternative system with alternative arrangement of said control unit 9 of the supervised device A is presented in fig. 2.

In order to supply the power supply voltages necessary for the operation of the device U the power supply module 1 is connected in such a way that its connector p1 is connected to the connector p2 of the pressure sensor 4, the connector p2 is connected to the connector p2 of the data analysis module 3, the connector p3 is connected to the connector p1 of the time standard and time synchronization module 2, while the connectors p4 and p5 of the power supply module 1 are connected to the connectors p1 and p2, respectively, of the control module 9 of the supervised device A. The pressure sensor 4 is connected through its connector p1 to the connector p1 of the data analysis module 3 in order to transmit a signal that reflects the actual pressure at the mouthpiece port 12. The connector p2 of the time standard and time synchronization module 2 is in turn connected to the connector p3 of the data analysis module 3 in order to transmit the time standard signals. The connector p4 of the data analysis module 3 is connected to the connector p3 of the control module 9 of the supervised device A in order to send control signals. The connectors p5 and p6 of the data analysis module 3 are connected to the connectors p1 and p2 of the data transmission and acquisition module 6 in order to send and receive signals. The connector p7 of the data analysis module 3 is connected to the connector p1 of the signal module 5 in order to send control signals. The connector p3 of the data transmission and acquisition module 6 is connected to the connector p2 of the signal module 5 in order to transmit signal about the status of using of the connection module 8, preferably in the form of an USB port.

In order to administer of a medicament, before starting of generation of aerosol, data describing parameters of the nebulisation method selected to the supervision are recorded to appropriate data storage media in the range of: the dose expected as the dose received by a patient, the concentration of the medicament used and its volume, the volume of other fluids used, are recorded to the appropriate data storage media. In addition, the data describing parameters of the device A selected to the supervision for using nebulisation method are recorded on the appropriate data storage media in the range of: the air volume generated per unit of time and an output of the liquid phase consumed per unit of time to produce an aerosol, as well as data describing an aerosol volume, which contains the dose equal to the desired dose, which is the pre-set dose, and the time of generation of an aerosol required to disperse the desired dose characterized for a specific type of the device A and specific therapeutic method.

All the recorded data are then used to appropriate calibration of the device A used as well as of the unit for medicament administering by the inhalation method, taking into account the parameters of the medicament, and for determining both a value of the threshold aerosol volume, in which a medicament dose that should be administered, i. e. pre-set dose, is contained, as well as a time needed for supplying the whole pre-set dose, i.e. threshold value of the duration time of an the aerosol generation and the suitable setting of the data analysis module 3.

A solution of the therapeutic substance is prepared in a volume containing the desired dose and a preselected inhalation device A of any suitable type for an aerosol generation and for administration of this substance is prepared according to their properties, purpose and above described parameters.

The device U of the present invention is designed to cooperation with any inhalation device A after suitable connection and it can control its operation and supervise an aerosol generation process during metering a dose of the pre-set substance in real time throughout the full metering process.

Device of the invention operates in four modes: in the standby mode, in the mode of diagnosis and optimization of the selection of therapeutic methods, in the mode of reproduction in laboratory conditions of the inhalations performed in the past and in the supervision mode of the devices for performing inhalations and monitoring the substance dose which they release.

The operation in the standby mode:

At a time when the device is not used, only three modules operate: a the power supply module 1, the time standard and time synchronization module 2, and the data analysis module 3. The data analysis module 3 and the time standard and time synchronization module 2 is supplied from DC battery permanently arranged in the power supply module 1. In this mode, data analysis module 3 operates in a low power consumption mode only in order to maintain a function of updating of the real time, by counting and processing cycles generated by the time standard and time synchronization module to date format and updates on ongoing basis date, hour, minute and second recorded in its FLASH memory.

The operation in the mode of diagnosis and optimization of the selection of therapeutic methods, including operation with dry powder inhalers:

In this operation mode, the device of the invention retains its functionality in the range of measurement and analysis of phenomena occurring in the nebuliser without having to physically connect the complete device for nebulisation. In order to use the device in the mode of diagnosis and optimization of the selection of therapeutic methods it is connected via the connection unit 8, in the preferred embodiment in the form of an USB port, to an external device, preferably to a processor or computer. The device U is usually supplied through USB port with 5V voltage that comes from an external device. In that situation all modules except the control module 9 for controlling of external devices are supplied. It is possible to perform measurements of pressure changes in the nebuliser which is not a part of the nebulisation device, that is, it is possible to carry out simulation of the procedure without the use of medicaments or other elements of the device to conduct inhalation. The aim of such approach is to create opportunities to test multiple virtual models of devices without having to administer medicament before the final choice of one of them and use it in reality. By measuring the maximum vacuum which a patient can produce it is possible to analyse the applicability of dry powder inhalers for this patient.

The operation in the mode of the control and supervision of a device for performing inhalation and monitoring the substance dose which it releases:

It is selected any device for inhalation in the form of an inhaler comprising nebuliser or ultrasonic inhaler or similar type inhaler, of continuous or electronically controlled operation, the parameters of which, describing a flow rate of fluid per unit time and a volume of aerosol produced per unit of time, are known.
It is selected any therapeutic method with a specific medicament dose, which should be administered, of given medicament concentration used in this method, having known used volume of a medicament and a given volume of diluent. Medicament substance in a form of a medicament or a mixture of medicament and diluent is prepared in accordance with selected method and it is poured into the nebuliser of the selected device A or into the appropriate chamber of the ultrasonic inhaler of the device A or other suitable type inhaler.

On the basis of significant parameters of the therapeutic method and significant parameters of the device A to the application of the method such as: the applied medicament concentration, the volume of medicament used, the volume of diluent used, the output per unit of time of a device A used for inhalation, the volume of the aerosol produced per unit time by the inhalation device used, preferably inhaler, it is calculated, using any suitable algorithm, the volume of the aerosol, in which a dose that should be administered is contained, in accordance with the accepted therapeutic method, hereinafter the pre-set dose. Separately, it is calculated in any way the time in which the applied (currently used) specific selected device A produces the volume of the aerosol containing the pre-set dose, which should be administered to the patient's respiratory track.

The following are examples of algorithms that can be used to calculate the threshold value of the aerosol volume, in which the pre-set dose is contained and the threshold value of time, in which the pre-set dose of the medicament in the given device for inhalation will be generated:

The exemplary algorithm to calculate the volume of the aerosol containing the known expected dose whereas the other parameters of the medicament and supervised device are known:

$$expected\ volume\ ml = \left( \frac{expected\ dose\ mg * flow\ rate\ \frac{ml}{min}}{\frac{medicament\ concentration\ \frac{mg}{ml} * medicament\ volume\ ml * flow\ rate\ \frac{ml}{min}}{medicament\ amount\ ml + diluent\ amount\ ml}} \right)$$

The above exemplary algorithm can be written also in a linear version as follows:

expected volume = (expected dose (mg * flow rate ml/min)/(((medicament concentration mg/ml * medicament volume ml * flow rate ml/min)/(medicament amount ml + diluent amount ml)))

The exemplary algorithm to calculate the time value for generation of the aerosol containing the known expected dose, i.e. the pre-set dose whereas the other parameters of the medicament and inhalation device are known:

$$expected\ time\ min = \left( \frac{expected\ dose\ mg}{\frac{medicament\ concentration\ \frac{mg}{ml} * medicament\ amount\ ml * flow\ rate\ \frac{ml}{min}}{medicament\ amount\ ml + diluent\ amount\ ml}} \right)$$

The above exemplary algorithm can be written also in a linear version as follows:

expected time min = (expected dose mg)/(((medicament concentration mg/ml * medicament amount ml * flow rate ml/min)/(medicament amount ml + diluent amount ml)))

The expected dose mentioned above corresponds to the pre-set dose.

Next it is determined which of these values, i.e. either the value that is obtained preferably by auto-calibration of the device or the value obtained at on the basis of reading the value resulting from the breath characteristics of the given patient, recorded on the data storage media, is the threshold pressure value below which the device of the invention will recognize an inspiration phase. The above mentioned values are recorded by any known method on electronic data storage medium, preferably in the form of a data file.

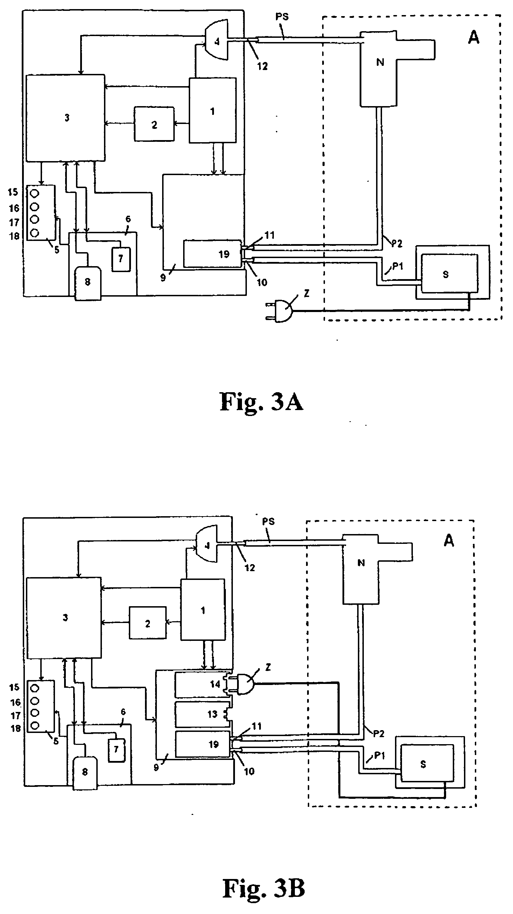

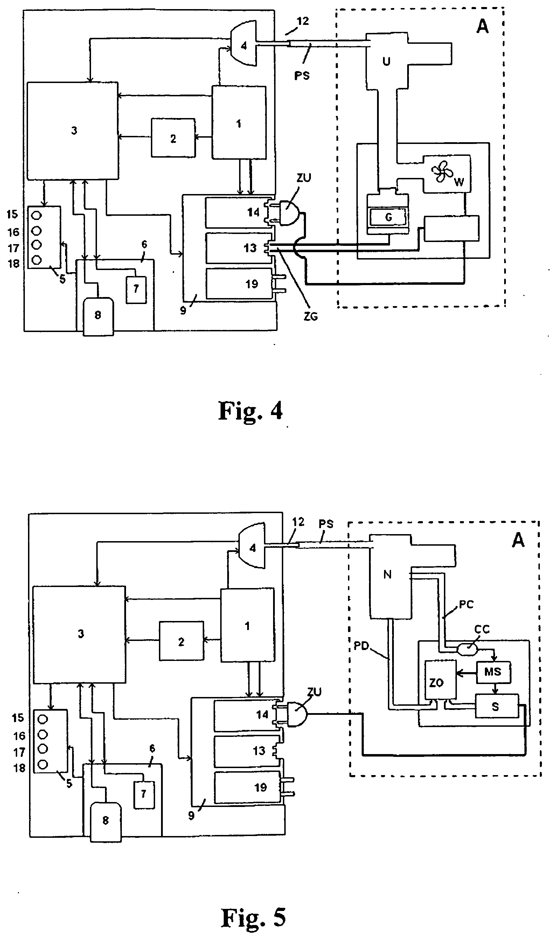

In a preferred embodiment of the invention, appropriate for use with most commonly used on the market inhalation devices using compressed air, which are not equipped with electronic control to its delivering, the device of the invention is combined with the given supervised inhalation device A in accordance to the connection diagram shown in fig. 3a or optionally in fig. 3b. In an alternative preferred embodiment, in the case of an ultrasonic nebuliser, the connection diagram shown in fig. 4 is preferably used. In an alternative preferred embodiment, in the case of the device equipped with an electronic air cut off system, the connection diagram shown in fig. 5 is preferably used.

The supervised device A for using of the inhalation method, in the preferred embodiment of the invention in a form of a nebuliser N, which is cut off from an air source at the valve 19 of the control module 9 for controlling the supervised device and it does not produce an aerosol, is turned on. In an alternative preferred embodiment, in the case of the inhalation device A equipped with an electronic air cut off system, a power supplying is cut off on the socket 14, in yet another alternative preferred embodiment, in the case of inhalation device in the form of ultrasonic nebuliser the power supplying of the ultrasonic probe is disconnected on the socket 13 of the device A U and aerosol is not produced.

In a basic preferred embodiment, the device U according to the invention is started through switching the electric power supply on, preferably from a separate DC power supply source of 12V voltage. In a preferred alternative embodiment by using an optional AC/DC converter the invention can be powered from normal mains with 230V/50Hz AC electric current. The non-converted voltage 230V AC reaches the connector p2 of the control module 9 for controlling the operation of external devices via the connector p5 of the power supply module 1. The converted and stabilized voltage of 5V DC is delivered to the connectors p1, p2 and p3 of the power supply module 1, while the converted and stabilized voltage 12V DC is delivered to the connector p4 of the power supply module 1, and then to the connector p2 of the control module 9 for controlling the operation of external devices.

The time standard and time synchronization module 2 transmits signals from its connector p2 to the connector p3 of the data analysis module 3, synchronizing the course of the phenomena occurring during proceeding the inhalation in time. The signal module 5, comprising signal diodes, preferably LEDs 15, 16, 17, receives a signal to its connector p1 from the connector p1 of the data analysis module 3 and preferably switches on the LED 15 informing about the fact that the power supply is turned on. The first step performed before starting operation is auto-calibration of the pressure sensor 4. Auto-calibration is carried out in such a way that with the data analysis module 3, through preferably 100 single cycles, forming a series of measurements, defined by the time standard and time synchronization module 2, the measurement of the voltage value transmitted to the connector p1 of the module 3 from the connector p1 of the pressure sensor 4 is carried out and the an average value of reading of a series of measurements of that voltage is recorded as the threshold pressure value in the FLASH memory of the data analysis module 3. The averaged value of the voltage on the connector p1 of the data analysis module 3 obtained in standby conditions is treated as a threshold value of the measured pressure at the port 12 and it is a reference value for subsequent measurements. In the next step the data analysis module 3 resets the value of the aerosol volume used to the application of the given therapeutic method, it resets the time used for the purpose of the application of the given therapeutic method and it storages these values in its FLASH memory. The data analysis module 3 awaits a signal from the communication and data acquisition module 6. The communication and data acquisition module 6 checks the status of the communication channel preferably via USB 8. In the case of the status where the USB port 8 is not currently used for communication to external devices such as disconnected USB cable/cord, it generates signal on the connector p1 and sends it to the connector p6 of the data analysis module 3. In response to this signal the data analysis module 3 goes into the analysis mode using pre-defined data, i.e. pre-set ones, recorded in its FLASH memory located in the data analysis module 3, containing standardized data preferably of the aerosol volume 10L/min, preferably the flow rate of the liquid phase 1ml/min, preferably the medicament content in the liquid phase of 100%, preferably medicament concentration 1mg/ml, preferably the aerosol volume, in which a dose equal to the desired dose, preferably 10 L, is contained, and the time required to generate the aerosol volume containing the desired dose that is the pre-set dose, preferably about 1 min. Such standardized data are loaded into the device during its manufacturing phase. The threshold pressure value is the value calculated in the auto-calibration process.

In the case when the communication and data acquisition module 6 finds a status in which the USB port 8 is currently used for communication to an external device (i.e. the USB cable/cord is connected), it sends a signal from the connector p3 to the connector p2 of the signal module 5, which preferably switch the LED 16 on which informs about the establishment of the connection through the communication channel. From the connector p1 of the communication and data processing module 6 the signal is transmitted to the connector p6 of the data analysis module 3, which informs about the establishment of the connection to an external device, which allows to start mutual communications between the device U according to the invention and an external device.

In response to a signal from an external device, which is the command to read archival data, the data analysis module 3 opens the connection between its connectors p6 and p5 and the connector p2 of the communication and data acquisition module 6, which allows to perform transmission of the data recorded on the data storage device 7, preferably on the micro SD card via USB port 8. When the signal appears constituting a command to download data the data analysis module 3 goes into the standby mode to record the external data. The values describing the selected method and the selected inhalation device A, connected to the device U according to the invention, which will be supervised, are loaded via the USB port 8 and the data transmission and acquisition module 9 through recording into the FLASH memory of the data analysis module 3 of the aerosol volume, in which a dose equal to the desired dose, i.e. the pre-set dose in litres, is contained, the time required to generate the aerosol volume containing the desired dose, i.e. the pre-set dose, in minutes, and the threshold pressure value for the pressure sensor.

These values correspond closely to the specific statement of the aerosol volume in L/min, generated by the supervised inhalation device A, for example the nebuliser, connected to the device U according to the invention, to the flow rate of the liquid phase in ml/min of this inhalation device A, to the original concentration of the medicament in mg/ml before possible dilution, to the compactness of the medicament in the liquid phase in mg/ml after possible dilution, and to the volume of the liquid used in the given therapeutic method. The threshold value for the pressure sensor 4 obtained from the data file is compared with the value calculated during the auto-calibration process

Finally, the lower value from the comparable values is preferably adopted.

This solution allows controlling the threshold of sensitivity of the pressure sensor, which in turn allows controlling both, delay of beginning of administration of the aerosol, as well, as acceleration of the moment of finishing of the aerosol administration. The external communications and data acquisition module 6 and the data analysis module 3 stop handling of data retrieval. The external communications and data acquisition module 6 goes into a standby mode to transmit/record data and waits for a signal from the data analysis module 3. The data analysis module 3 is ready for evaluation and interpretation of the signal status from the pressure sensor 4. The control module 9 for controlling of the supervised device goes into a standby characteristic with the valve 19 in its closed position, or in another preferred embodiment with the optionally switched off power supply of the ultrasonic probe 13, in the case of application of the ultrasonic inhaler. In one embodiment of the invention, the socket 14 supplies the supervised device A, using electronic cutting off of the air flow, which operate independently of the operation of the device according to the invention. The device U of the invention is at that moment ready for operation and the inhalation device A for administering aerosol is ready for operation.

The above described activities constitute the initial calibration and adjustment step of the device according to the invention to operation with the (specific) inhalation device, to which it was attached, and with the given medicament to be administered by the inhalation device. The device of the invention together with an inhalation device are connected at this stage of the operation in one system the further operation of which is dependent on Individual patient breathing pattern, supported by the inhalation device and which is controlled by the patient's breath. As long as the patient "sucks" aerosol, i.e. generates a pressure lower than the threshold pressure value, we are sure that he/she receives the whole generated aerosol. When an amount of the aerosol is "too much" - i.e. either the supervised device has too much capacity in relation to the capacity of the patient's respiratory track, for example of a child, or the inspiration phase of the patient ends, and together with it the ability to continue "sucking", the pressure cannot be lower - the device U detects and reads the pressure increase. In this situation, the device U of the invention automatically causes automatic closing of the valve 19, and the production of aerosols ends.

Since the moment when the device U according to the invention is ready to operation, the signal status of the pressure sensor 4 is sampled by the data analysis module 3 using the regime determined by the time standard and time synchronisation module 2. The pressure drop in the signal cable connecting the nebutiser, i.e. the inhaler device A for metering a dose of the substance and the pressure sensor 4 of the device U of the invention by the port 12 is recognized and interpreted as a value below the threshold value and it means beginning of inspiration. A single measurement cycle determined through the time standard and time synchronization module 2 is started. The data analysis module 3 uses the retrieved value of the signal received from the pressure sensor 4 for calculating the instantaneous values of the volume of aerosol produced by the supervised device A in close conjunction with its characteristics. This value, together with the used control signal generated by the data analysis module 3 within the time frame specified by the signal generated by the time standard and time synchronization module 2 is sent to the external communication and data acquisition module 6, where in the preferred embodiment, it is transmitted by the connecting device 8, for example, a USB port for any use and preferably simultaneously recorded on a data storage device, for example, on a micro SD card 7. In a preferred embodiment, the signal of the transmitted data is used for the current graphical presentation of the value processed by the data analysis module 3. Until the time of recognition of the first inspiration, both the aerosol volume used to metering the pre-set dose and the time consumed for metering the dose have zero values. The data analysis module 3 adds the created value of the instantaneous aerosol volume to the value of the aerosol volume used and then it compares the resulting summary value to the threshold value, described as the volume of the aerosol, in which a dose equal to the pre-set (desired) dose is contained. Analogically, the data analysis module 3 adds the instantaneous time of the aerosol generation to the time used, and then compares the summary value obtained to the total time required to disperse the pre-set dose or the desired dose and waits for the end of a single measurement cycle. The single measuring cycle determined by the time standard and time synchronization module 2 is stopped. If the values of the volume used and the time taken are lower than the pre-set and calculated threshold values (see algorithms), the data analysis module 3 is waiting for another signal from the time standard and time synchronization module 2, and repeats the single measurement cycles described until the time at which the signal value of the sensor 4 does not reach again the threshold or higher value, or the volume of the aerosol consumed to the application of the dose, which is a sum of the instantaneous volumes measured in single measurement cycles, is greater than the value of the volume described as the aerosol volume, in which a dose equal to the pre-set dose is contained, or the time used effectively for metering the dose, which is the sum of the instantaneous times for the aerosol generation, measured in single measurement cycles, is greater than the value specified by the total time required to disperse the pre-set dose. The operation of a nebuliser or other device connected to the device U of the invention will stop after fulfilling of any first of the above conditions, wherein both these conditions, i.e. both exceeding of the threshold value of the volume of the aerosol generated, as well as the threshold value of the time of the generation of the aerosol are equivalent, however the physically and truly measured value is the time, especially its intervals or cycles, while the volume of the aerosol is the value calculated in each of these cycles. It may happen that due to inaccuracy of the formulas (e.g. rounding up), exceeding of the calculated values of the volume will appear first and it "overtakes" exceeding the value of the time, but it will be safe for the patient bias, because in reality, the amount of the administered aerosol should be less than the amount of the calculated aerosol and therefore it is important to both parameters, the value of the volume of aerosol and value of time for the aerosol generation, were monitored and used as a second protection, in this way the double protection is used in the device U in order to ensure generation and delivering the appropriate pre-set dose to the patient's respiratory track. Up to the moment of exceeding of the pre-set, calculated threshold values of the aerosol volume and/or duration time of the aerosol generation for delivering the pre-set dose, regardless of the number of individual measurement cycles, the data analysis module 3 sends an appropriate signal to the signal module 5, which preferably switch the LED 17 on, which informs of the fact that the inspiration continues and to the control module 9 for controlling of the operation of the supervised device, which opens the connection at the valve 19 between the input port 10 and the exit port 11, allowing the flow of the air from the compressor connected to the device U according to the invention, to the supervised device A, preferably a nebuliser or other type of the device for inhalation, which is subject to control and monitoring, i. e. supervision, allowing to perform inhalation. In alternative preferred embodiments, performing of the inhalation allows supporting of the power supplying of the device for inhalation, i.e. the supervised device A, at the socket 14 of the device U of the invention, or switching on, at the socket 13 of the device according to the invention, the power supply of an ultrasonic probe G of the supervised device A for inhalation.

Since the data analysis module 3 detects the signal from the pressure sensor 4 of a value equal to or greater than the threshold pressure value, the device U of the invention recognizes the patient's exhalation. A single measurement cycle determined through the time standard and time synchronization module 2 is started. The processed signal values of the pressure sensor 4 are sent through the external communication and data acquisition module 6, preferably via the USB port 8, and are simultaneously recorded on a data storage device, preferably on a micro SD card 7. However, they are not counted as the volume used or the time taken for the effective medicament dose metering. A single measurement cycle determined through the time standard and time synchronization module 2 is finished. The data analysis module 3 awaits the next signal from the time standard and time synchronization module 2, and repeats the described single cycles until the signal value of the pressure sensor 4 reaches again or is lower than the threshold pressure value. Until then, the data analysis module 3 sends an appropriate signal to the signal module 5, which preferably switches the LED 17 off and suppresses information about the fact of continuation of the inspiration and to the control module 9 for controlling operation of the supervised device, which closes the valve 19 and stops the production of the aerosol. In alternative preferred embodiments the power supply socket 13 of the ultrasonic probe of the inhalation device is switched off, in order to stop production of the aerosol during ineffective time (exhalation). Devices equipped with their own electronic control system for aerosol administration are still powered through the socket 14 and operate independently of the device U according to the invention.

When, during inspiration, in any single measurement cycle it is found that the aerosol volume used is equal to or exceeds the volume described as the volume of the aerosol, In which a dose equal to the pre-set/desired dose is contained, or the time taken is equal to or exceeds the value specified by a total time required to disperse the pre-set dose, the data analysis module 3 stops sampling of the status of the signal of the sensor 4, it does not process it and it does not react to its changes, while also sends signals: to the external communication and data acquisition module 6, to the control module 9 for controlling operation of the supervised device and to the signal module 5. The communication and data acquisition module 6 ends communication through the USB port 8 and recording on data storage device, such as a micro SD card 7, the control module 9 for controlling operation of the supervised device prevents further generation of the aerosol by closing the valve 19 in one embodiment, and in other embodiments by switching off the power supplying of sockets 13 and 14, the signal module 5 switches the LEDs 16 and 17 off and preferably switches on the LED 18, i.e. displays information about completion of the process of administering and metering aerosol dose. Further operation of the device according to the invention and the supervised inhalation devices A connected to it is not possible.

Fig. 1 shows schematically, according to the above description, the controlling and monitoring device U according to the invention of the device for inhalation in the first embodiment Fig. 2 shows a schematic diagram of the device of the invention in another embodiment equipped with an expanded control module 9 for controlling operation of the supervised device A.

According to an embodiment the device U according to the invention is combined into single unit with the appropriate selected supervised inhalation device A, which comprises at least the power cord Z of the compressor S, the nebuliser N and the pressure lines P1, P2, which are connected to the device of the invention in such a way that the stream of the compressed air from the compressor S of the device A is directed by means of the segment of the pressure fine P1 arranged proximal to the inhalation device A side to the input port 10 of the device U according to the invention in the control module 9 for controlling operation of the supervised device A. The output port 11 of this module is connected by means of the pressure line P2 to the nebuliser N of the supervised device A. The nebuliser of the inhalation device A is connected by means of the signal cable PS to the port 12 of the pressure sensor 4 of the device according to the invention.

Fig. 3a shows schematically the manner in which the device U according to the invention is connected to commonly used devices for generation of the aerosol by the pressure method, without the electronic control of aerosol production.

Fig. 3b shows schematically the manner in which the device U according to the invention is optionally connected to commonly used supervised devices A for generation of the aerosol by the pressure method, without the electronic control of the aerosol production. According to this solution the device A configured as shown in fig. 3a, is additionally connected to the device U according to the invention in such a way that the power cord Z of the supervised device A is connected to the socket 14 of the device U of the invention in the control module 9 for controlling operation of the supervised devices.

Fig. 4 shows schematically another variant of the method for connecting the device U according to the invention to the supervised devices A for generation of aerosol by ultrasonic method. The supervised device A according to this embodiment comprises at least the power cord ZU for power supplying of the device, an ultrasonic probe G supplied by the power cord ZG for supplying the probe, the fan W for forcing the movement of the aerosol, and the mouthpiece U. The mouthpiece U of the device connected to the supervised device A is connected by means of the signal cable PS to the port 12 of the pressure sensor 4 in the device according to the invention. Modified power cord ZG for power supplying of the ultrasonic probe G is connected to the socket 13 of the control module 9 for controlling operation of the supervised device of the invention. However, the main power cord ZG of the supervised device A is connected to the socket 14 of the control module 9 for controlling operation of the supervised device of the invention.

Fig. 5 shows schematically the method of connection of the device A for nebulisation by pressure method, of the type having its own control system for controlling generation of aerosol, being the supervised device A, to the unit U according to the invention. In this embodiment the supervised device A comprises at least the power cord ZU for power supplying of the device, the compressor S, the nebuliser N and the cable PD for delivering compressed air to the nebuliser N and it is additionally equipped with a pressure sensor CC, a control module MS and a shut-off valve ZO. This type of the device A is connected by plugging the power supply cord ZU of the supervised device A into the socket 14 of the control module 9 for controlling operation of the supervised device of the invention.

## Claims

1.  A device (U) for controlling and monitoring of a supervised device (A) for generation of an aerosol during metering of a medicament dose, in particular for supervision of a process of generation of an aerosol during metering of administered medicament dose defined as a pre-set dose, and especially of a supervised inhalation device (A) employed for continuous generation of an aerosol comprising a mouthpiece, which device (U) is configured to cooperate with said supervised device (A) and can be connected to said supervised device (A) that is in the form of:

    - an inhaler for generation of an aerosol by pressure method comprising at least a power cord (Z), a nebulizer (N), a compressor (S) and,
    when it is of the type without an electronic control of an aerosol production further having pressure lines P1,P2, or when it is of the type having its own control system for controlling generation of an aerosol further having a power cord (ZU);
    or
    - a ultrasonic inhaler for generation of an aerosol by ultrasonic method having a ultrasonic probe (G) and a power supply cord (ZG) of said probe (G),
    and the device (U) comprising:

        a power supply module (1),
        a pressure sensor (4) with a port (12),
        a signal module (5),
        a data analysis module (3) including a FLASH memory in it,
        a data transmission and acquisition module (6) with a connection unit (8), preferably USB port (8),
        a time standard and time synchronization module (2) transmitting signals to said data analysis module (3), that are time standard signals, during proceeding the inhalation in time and determining single measurement cycle and
        a control module (9) for controlling said supervised device (A), that module (9) is further provided with:

            - connection sockets (13, 14) for connection to main supply line that is said power cord (ZU) and/or said power cord (ZG) of the probe (G)
            - at least one valve (19), preferably electric valve (19) with an output port (11) and an input port (10), at said valve (19) there is connection between the input port (10) and the output port (11), wherein pressure lines (P1) and (P2) can be connected to the device (U) so that, the stream of compressed air from compressor (S) is directed by said pressure line (P1) to the input port (10) in the control module (9) and the output port (11) of this module (9) is connected by said pressure line (P2) to the nebulizer (N),

wherein in use, during metering medicament dose, the device (U) together with said supervised inhalation device (A) are connected at this stage of operation in one system, the further operation of which is dependent on individual patient breathing pattern and which is controlled by a patient breath, and further wherein the device (U) is configured:

- to perform initial calibration and adjustment step of the device (U) to operation with said specific supervised inhalation device (A), to which it is attached, during which step values describing a selected method and a selected inhalation device (A) connected to the device (U) which is supervised are loaded via said USB port (8) and said data transmission and acquisition module (6) through recording into the FLASH memory of said data analysis module (3) of an aerosol volume, in which a dose equal to the pre-set dose in litres is contained, a time required to generate an aerosol volume containing the pre-set dose in minutes, and threshold pressure value for said pressure sensor (4), and
- to perform an individual calibration for each said system formed with determining its:

- individual threshold pressure value for the pressure sensor (4) below which the device (U) recognizes an inspiration phase, which value is interpreted as beginning of inspiration phase and exhalation phase; and
- threshold aerosol volume value, as well as,
- threshold time value of duration of aerosol generation,

- to control operation of said supervised device (A), and wherein during metering said dose a pressure drop below the threshold pressure value is detected, which means start of inspiration phase and start of generation aerosol by said supervised inhalation device (A), which generation is continued until the moment of reaching again of the threshold pressure value or higher value, that is interpreted as beginning of exhalation phase, and wherein a process of generation of an aerosol during metering of a dose in supervised device (A) is completed at the time when a volume of aerosol used for metering of a dose, which is a sum of instantaneous volumes measured in single measurement cycles, exceeds the threshold value of an aerosol volume described as such a volume of an aerosol, in which a dose equal to the pre-set dose is contained and/or when a time used effectively for generation of an aerosol during metering of the dose, which is a sum of instantaneous times of generation of an aerosol, measured in single measurement cycles, exceeds the threshold time value of generation of said aerosol, determined by total time required to disperse the pre-set dose.

2.   The device (U) according to claim 1 **characterized in that**, the power supply module (1) of the controlling and monitoring device (U) for power supplying is connected to the pressure sensor (4), as well as to the data analysis module (3) and to the time standard and time synchronization module (2), while its other connections are connected to connections of the control module (9) for controlling the supervised device (A), respectively, while the pressure sensor (4), transmitting a signal reflecting a current pressure at the signal cable (PS) connecting supervised inhaler device (A) and the pressure sensor (4) by the port (12) is connected to the data analysis module (3), to which the time standard and time synchronization module (2) is also connected, which module (3) sends control signals, via and adequate connection, to the control module (9) for controlling said supervised device (A), wherein the data analysis module (3), in order to send and receive signals, is also connected to the data transmission and acquisition module (6) and the signal module (5), respectively, to which the data transmission and acquisition module (6) has also separate connection in order to transmit signal on a status of using of the connection unit (8), preferably in the form of USB port (8).

3.   The device (U) according to claim 1 or 2, **characterized in that** the signal module (5) comprises signal diodes, preferably LEDs (15, 16, 17, 18), of which the LED (15) indicates a power supply connection, the LED (16) indicates a connection to the communication and data transmission module (6), while the LED (17) indicates an inspiration and metering phase, and the LED (18) indicates stopping of metering.

4.   The device (U) according to at least one of claims 1 or 2 or 3, **characterized in that** it is connected to said supervised device (A) in such a way that

- the port (12) of the pressure sensor (4) of the controlling and monitoring device (U) is connected to the signal cable (PS) connecting by the port (12) said supervised inhaler device (A) to the pressure sensor (4) of the controlling and monitoring device (U) and outgoing from the nebulizer (N) or from a mouthpiece (US) of the supervised device (A); and

- the power cord (ZU) of said supervised device (A) is connected to a corresponding connection of the control module (9) for controlling operation of the supervised device, namely: a socket (14), and/or

- the power cord (ZG) of an ultrasonic probe (G) is connected to the socket (13) of the control module (9) for controlling operation of supervised device (A); and/or

- the pressure line (P1) outgoing from the compressor (S) of said supervised device (A) is connected to the input pressure ferrule (10) of the control module (9), while the pressure output port (11) of the control module (9) is connected by the pressure line (P2) to the nebuliser (N) of said supervised device (A).

## Patentansprüche

1. Vorrichtung (U) zur Steuerung und Überwachung eines zu überwachenden Geräts (A) zur Aerosolerzeugung bei der Applikation einer Arzneidosis, insbesondere zur Überwachung des Aerosolerzeugungsvorgangs bei der Applikation einer als Soll-Arzneidosis bezeichneten Arzneidosis, insbesondere eines zu überwachenden Inhalationsgeräts (A) zur kontinuierlichen Aerosolerzeugung, welches ein Mundstück umfasst, welche Vorrichtung (U) eingerichtet ist, mit dem genannten zu überwachenden Gerät (A) zusammenzuwirken, und welche mit dem genannten zu überwachenden Gerät (A) verbindbar ist, wobei das Gerät (A) als:

- ein zumindest eine Versorgungsleitung (Z), einen Nebuliser (N) und einen Verdichter (S) umfassenden Inhalationsgerät zur Aerosolerzeugung mittels Druckverfahren, das ferner
für den Fall, dass dieses über keine elektronische Steuerung der Aerosolerzeugung verfügt, Druckleitungen (P1, P2) aufweist bzw.
für den Fall, dass dieses über eine eigene Steuerung der Aerosolerzeugung verfügt, eine Versorgungsleitung (ZU) aufweist; oder
- ein einen Ultraschallkopf (G) sowie eine Kopfversorgungsleitung (ZG) dieses Ultraschallkopfs (G) umfassenden Ultraschallinhalationsgerät zur Aerosolerzeugung mittels Ultraschallverfahren,
ausgebildet ist,
und die Vorrichtung (U):

einen Versorgungsmodul (1),
einen Drucksensor (4) mit einem Anschluss (12),
einen Signalmodul (5),
einen Datenverarbeitungsmodul (3) mit einem darin enthaltenen Flash-Speicher,
einen Datenübertragungs- und Datenerfassungsmodul (6) mit der Anschlusseinheit (8), vorzugsweise in Form von einem USB-Port,
einen Zeitnormalisierung- und Zeitsynchronisierungsmodul (2), der auf den genannten Datenverarbeitungsmodul (3) Signale überträgt, die die Zeitnormalisierungssignale während der Inhalationsführung darstellen und einen einzelnen Messzyklus bestimmen, sowie
einen Modul (9) zur Steuerung des zu überwachenden Geräts (A), welcher Modul ferner mit:

- Anschlussaufnahmen (13, 14) zur Verbindung mit der Versorgungshauptleitung, die die genannte Versorgungsleitung (ZU) und/oder die Kopfversorgungsleitung (ZG) des Ultraschallkopfs (G) ist,
- mindestens einem Ventil (19), vorzugsweise einem elektrischen Ventil (19) mit dem Auslassanschluss (11) und dem Einlassanschluss (10), versehen ist, wobei die Druckleitungen (P1) und (P2) an die Vorrichtung (U) derart angeschlossen werden können, dass der Druckluftstrom aus dem Verdichter (S) durch die genannte Druckleitung (P1) an den Einlassanschluss (10) im Steuerungsmodul (9) und der Auslassanschluss (P2) dieses Steuerungsmoduls (9) durch die genannte Druckleitung (P2) an den Nebuliser (N) angeschlossen ist,

umfasst,
wobei die Vorrichtung (U) bei der Verwendung zur Applikation einer Arzneidosis und das genannte zu überwachende Inhalationsgerät (A) in diesem Arbeitsschritt miteinander zu einer Einheit verbunden sind, deren Funktionsweise anschliessend von dem individuellen Atemmuster des Patienten abhängt und die mit dem Atem des Patienten steuerbar ist,
wobei ferner die Vorrichtung (U) eingerichtet ist:

- den Schritt der Vorkalibrierung durchzuführen und die Vorrichtung (U) an die Zusammenwirkung mit dem genannten zu überwachenden Inhalationsgerät (A) anzupassen, an das die Vorrichtung (U) angeschlossen wurde, bei welchem Schritt die das ausgewählte Verfahren und das ausgewählte zu überwachende, an die Vorrichtung (U) angeschlossene Inhalationsgerät (A) beschreibenden Werte durch den USB-Port (8) und den Datenübertragungs- und Datenerfassungsmodul (6) heruntergeladen werden, derart, dass im Flash-Speicher des Datenverarbeitungsmoduls (3) ein Aerosolvolumen, in dem eine der Soll-Dosis entsprechende Dosis in Liter enthalten ist, die zur Erzeugung des die Soll-Dosis enthaltenden Aerosolvolumens erforderliche Zeit in Minuten und den Druckschwellwert für den Drucksensor (4) eingetragen wird, und

- die individuelle Kalibrierung für die jeweilige genannte so gebildete Einheit durchzuführen, indem dafür individuell der Druckschwellwert für den Drucksensor (4), unter welchem die Vorrichtung (U) der Anfang der Inhalationssphase erkannt und welcher als der Anfang der Inhalationssphase sowie der Exhalationssphase interpretiert wird, sowie

- den Grenzwert des Aerosolvolumens, als auch
- den Grenzwert der Dauerzeit der Aerosolerzeugung ermittelt wird,

- den Betrieb des genannten zu überwachenden Gerät (A) zu steuern, sowie wobei während der Dosisapplikaton die Überschreitung des Druckschwellwertes erfasst wird, die dem Anfang der Inhalationssphase entspricht, und die durch das genannte zu überwachende Inhalationsgerät (A) auszuführende Aerosolerzeugung ausgelöst wird, welche fortgesetzt wird, bis der Druckschwellwert wieder erreicht oder überschritten wird, was als die Ausatmungsphase erfasst wird, wobei der Aerosolerzeugungsvorgang bei der Dosisapplikaton im zu überwachenden Inhalationsgerät (A) beendet wird, wenn das Volumen des erzeugten zur Dosissapplikations verwendeten Aerosols, das der Summe der momentanen in einzelnen Messzyklen gemessenen Volumen gleich ist, den Grenzwert des Aerosolvolumens überschreitet, die als der Aerosolvolumen definiert ist, in dem die der Soll-Dosis entsprechende Dosis enthalten ist, und/oder wenn die für die Aerosolerzeugung bei der Dosisapplikation effektiv benutzte Zeit, die der Summe der momentanen in einzelnen Messzyklen gemessenen Zeiten der Aerosolerzeugung gleich ist, den Grenzwert der Dauerzeit der Aerosolerzeugung überschreitet, die als die für die Dispergierung der Soll-Dosis erforderliche Gesamtzeit definiert ist.

**2.** Vorrichtung (U) nach Anspruch 1, **dadurch gekennzeichnet, dass** der die Versorgung bereitstellende Versorgungsmodul (1) der Vorrichtung (U) zur Steuerung und Überwachung mit dem Drucksensor (4) sowie mit dem Datenverarbeitungsmodul (3) und mit dem Zeitnormalisierung- und Zeitsynchroniserungsmodul (2) verbunden ist und seine andere Anschlüsse jeweils mit den Anschlüssen des Steuerungsmoduls (9) des zu überwachenden Geräts (A) verbunden sind, während der Drucksensor (4), der den jeweiligen Druck in der das zu überwachende Gerät (A) und den Drucksensor (4) durch den Anschluss (12) verbindenden Signalleitung (PS) wiedergebendes Signal übermittelt, mit dem auch mit dem Zeitnormalisierung- und Zeitsynchroniserungsmodul (2) verbundenen Datenverarbeitungsmodul (3) verbunden ist, welcher Steuersignale durch eine entsprechende Verbindung zum Steuerungsmodul (9) des zu überwachenden Geräts (A) übermittelt, wobei der Datenverarbeitungsmodul (3) zur Signalabgabe und - empfang ferner jeweils mit dem Datenübertragungs- und Datenerfassungsmodul (6) sowie mit einem Signalmodul (5) verbunden ist, mit welchem durch eine separate Verbindung auch der Datenübertragungs- und Datenerfassungsmodul (6) verbunden ist, um das Signal über den Ausnutzungszustand des Anschlussmoduls (8), vorzugsweise in Form von einem USB-Port, zu übermitteln.

**3.** Vorrichtung nach Anspruch 1 oder 2, **dadurch gekennzeichnet, dass** der Signalmodul (4) Signaldioden, vorzugsweise LED-Leuchtdioden (15, 16, 17, 18) umfasst, von denen die Diode (15) die Versorgung, die Diode (16) die Verbindung mit dem Datenübertragungs- und Datenerfassungsmodul (6), die Diode (17) die Inhalations- und Applikationsphase und die Diode (18) die Beendigung der Applikation signalisiert.

**4.** Vorrichtung nach mindestens einem der Ansprüche 1 oder 2 oder 3, **dadurch gekennzeichnet, dass** sie mit dem zu überwachenden Gerät (A) verbindbar ist, derart, dass

- der Anschluss (12) des Drucksensors (4) der Vorrichtung (U) zur Steuerung und Überwachung mit der Signalleitung (PS), die durch den Anschluss (12) das genannte Inhalationsgerät (A) mit dem Drucksensor (4) der Vorrichtung (U) zur Steuerung und Überwachung verbindet und aus dem Nebuliser (N) bzw. dem Mundstück (US) des zu überwachenden Geräts (A) herausgeführt ist verbindbar ist; sowie
- die Versorgungsleitung (ZU) dieses zu überwachenden Geräts (A) an den zugehörigen Anschluss, vorzugsweise die Aufnahme (14), des Steuerungsmoduls (9) des zu überwachenden Geräts angeschlossen ist; und/oder
- die Kopfversorgungsleitung (ZG) des Ultraschallkopfs (G) mit der Aufnahme (13) des Steuerungsmoduls (9) des zu überwachenden Geräts verbindbar ist; und/oder

- die aus dem Verdichter (S) des zu überwachenden Geräts (A) ausgehende Druckleitung (P1) mit dem Druckeinlassstutzen (10) des Steuerungsmoduls (9) verbindbar ist und der Druckauslassstutzen (11) dieses Steuerungsmoduls (9) durch die Druckleitung (P2) mit dem Nebuliser (N) des zu überwachenden Geräts (A) verbindbar ist.


**Revendications**

1.  Dispositif (U) de commande et de contrôle d'un dispositif supervisé (A) de génération de l'aérosol pendant le dosage d'une dose de médicament, en particulier de supervision du processus de génération de l'aérosol pendant le dosage d'une dose de médicament administré, définie comme une dose de consigne, en particulier d'un dispositif supervisé d'inhalation (A) de génération de l'aérosol en continu comprenant un embout buccal, lequel dispositif (U) est configuré pour coopérer avec ledit dispositif (A) supervisé et peut être relié au ledit dispositif (A) supervisé, qui a la forme :

    - d'inhalateur qui génère l'aérosol par méthode de pression comprenant au moins un tuyau d'alimentation (Z), un nébuliseur (N), un compresseur (S) et:

        quand il est de type sans commande électronique de génération de l'aérosol, comprenant en plus des conduites forcées (P1, P2), ou
        quand il est de type muni de son propre système de commande de génération de l'aérosol comprenant en outre un tuyau d'alimentation (ZU); ou

    - d'inhalateur à ultrasons pour générer l'aérosol par méthode à ultrasons comprenant une tête à ultrasons (G) et un tuyau d'alimentation (ZG) de cette tête (G),
    le dispositif (U) comprenant:

        un module d'alimentation (1),
        un capteur de pression (4) avec une connexion (12),
        un module de signalisation (5),
        un module (3) d'analyse des données à mémoire FLASH incorporée,
        un module (6) de transmission et d'acquisition de données avec un ensemble de connexion (8), de préférence sous forme d'un port USB,
        un module (2) d'étalon du temps et de synchronisation du temps, transmettant des signaux audit module (3) d'analyse des données, qui sont des signaux de standardisation du temps, au cours d'une inhalation dans le temps, et qui déterminent un cycle de mesure individuel, et
        un module (9) de commande du dispositif (A) supervisé, ledit module étant en plus muni :

    - de logements de connexion (13, 14) pour la connexion avec le tuyau d'alimentation principal, qui constitue ledit tuyau d'alimentation (ZU) et/ou le tuyau d'alimentation (ZG) de la tête (G),
    - d'au moins une vanne (19), de préférence une vanne électrique (19) à connexion d'admission (11) et à connexion d'évacuation (10), les conduites forcées (P1, P2) pouvant être connectées au dispositif (U) de telle manière que le flux d'air comprimé du compresseur (S) est dirigé par ladite conduite forcée (P1) vers la connexion d'admission (10) du module (9) de commande, et la conduite d'évacuation (P2) de ce module (9) de commande est reliée par ladite conduite forcée (P2) au nébuliseur (N),
    le dispositif (U), au cours de son utilisation, avec ledit dispositif d'inhalation (A) supervisé, au cours du dosage d'une dose de médicament, et à cette étape de fonctionnement, sont connectés formant un seul ensemble dont le fonctionnement dépend ensuite du mode de respiration individuel du patient et qui est commandé par la respiration du patient,

    ledit dispositif (U) étant en plus configuré :

    - pour réaliser l'étape de calibrage préliminaire et d'adaptation du dispositif (U) au travail avec ledit dispositif d'inhalation (A) supervisé, auquel il a été connecté, au cours de laquelle a lieu le téléchargement par le port USB (8) et le module (6) de transmission et d'acquisition des valeurs données de description du mode choisi et du dispositif d'inhalation (A) choisi, qui sera supervisé et qui est connecté au dispositif (U), par l'enregistrement dans la mémoire FLASH du module (3) d'analyse des données du volume de l'aérosol dans lequel est contenue la dose égale à la dose désirée en litres, le temps nécessaire pour générer le volume de l'aérosol contenant la dose désirée en minutes et la valeur de seuil de la pression pour le capteur de pression (4), et,

- pour réaliser un calibrage individuel pour chaque dit ensemble créé de cette façon, en désignant individuellement pour ce dernier la valeur de seuil de la pression sur le capteur de pression (4), au dessous de laquelle le dispositif (U) reconnaîtra le début de la phase d'inspiration, laquelle valeur étant interprétée en tant que début de la phase d'inspiration et de la phase d'expiration, ainsi que

- la valeur de seuil du volume de l'aérosol, et

- la valeur de seuil de la durée de la génération de l'aérosol,

- pour commander le fonctionnement dudit dispositif (A) supervisé, et,

au cours du dosage de la dose est effectuée la lecture du dépassement de la valeur de seuil de la pression, qui signifie le début de la phase d'inspiration, et on lance la génération de l'aérosol par ledit dispositif d'inhalation (A) supervisé, ladite génération étant continuée jusqu'au moment d'aboutir à nouveau à la valeur de seuil de la pression ou à une valeur supérieure à cette valeur, ce qui est lu en tant que phase d'expiration, et on termine le processus de la génération de l'aérosol à l'administration de la dose dans le dispositif d'inhalation (A) supervisé au moment où le volume de l'aérosol généré utilisé pour l'administration de la dose, constituant le total des volumes momentanés mesurés au cours des cycles de mesure individuels, est supérieur à la valeur de seuil du volume de l'aérosol décrite en tant que volume de l'aérosol dans lequel est contenue la dose égale à la dose désirée et/ou quand le temps effectivement utilisé pour générer l'aérosol à l'administration de la dose, constituant le total des durées momentanées de la génération de l'aérosol, mesurées au cours des cycles de mesure individuels, est supérieur à la valeur de seuil de la durée de la génération de l'aérosol, définie par la durée totale nécessaire à la dispersion de la dose désirée.

2. Dispositif (U) selon la revendication 1, **caractérisé en ce que** le module d'alimentation (1) du dispositif (U) de commande et de contrôle assurant l'alimentation est relié au capteur de pression (4), ainsi qu'au module (3) d'analyse des données et au module (2) d'étalon du temps et de synchronisation du temps, et **en ce que** ses autres connexions sont reliées respectivement aux connexions du module (9) de commande du dispositif (A) supervisé, pendant que le capteur de pression (4) transmettant le signal illustrant la pression actuelle dans la conduite de signalisation (PS) qui relie le dispositif d'inhalation (A) supervisé et le capteur de pression (4) par la connexion (12) est relié au module (3) d'analyse des données, auquel est également relié le module (2) d'étalon du temps et de synchronisation du temps, lequel module (3) d'analyse des données envoie des signaux de commande par l'intermédiaire d'une connexion appropriée vers le module (9) de commande du dispositif (A) supervisé, le module (3) d'analyse des données, pour envoyer et recevoir des signaux, étant en outre relié respectivement au module (6) de transmission et d'acquisition de données et au module de signalisation (5), avec lequel le module (6) de transmission et d'acquisition de données a également une connexion distincte, afin de transmettre un signal relatif à l'état d'utilisation du module de connexion (8), de préférence sous forme d'un port USB (8).

3. Dispositif (U) selon les revendications 1 ou 2, **caractérisé en ce que** le module de signalisation (5) comprend des diodes de signalisation, de préférence des diodes LED (15,16,17,18), dont la diode (15) signale le branchement de l'alimentation, la diode (16) signale la connexion avec le module (6) de communication et de transmission de données, la diode (17) signale la phase d'inspiration et de dosage, et la diode (18) signale la fin du dosage,

4. Dispositif (U) selon au moins une des revendications 1 ou 2 ou 3, **caractérisé en ce qu'**il est relié au dispositif (A) supervisé,
de telle manière que

- la connexion (12) du capteur de pression (4) du dispositif (U) de commande et de contrôle est relié à la conduite de signalisation (PS) qui relie par l'intermédiaire de la connexion (12) ledit dispositif d'inhalation (A) supervisé au capteur de pression (4) du dispositif (U) de commande et de contrôle et qui est menée du nébuliseur (N) ou de l'embout buccal (US) du dispositif (A) supervisé; et

- le tuyau d'alimentation (ZU) de ce dispositif (A) supervisé est relié à une connexion respective du module de commande (9) du comportement du dispositif supervisé, de préférence au logement (14) ; et/ou

- le tuyau d'alimentation (ZG) de la tête à ultrasons (G) est relié au logement (13) du module de commande (9) du comportement du dispositif (A) supervisé; et/ou

- la conduite forcée (P1) sortant du compresseur (S) du dispositif (A) supervisé est reliée au manchon d'admission de pression (10) du module de commande (9), et le manchon d'évacuation de pression (11) de ce module de commande (9) est relié par la conduite forcée (P2) au nébuliseur (N) du dispositif (A) supervisé.

**Fig. 1**

**Fig. 2**

**Fig. 3A**

**Fig. 3B**

**Fig. 4**

**Fig. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6606989 B
- US 6606989 B1
- DE 19939417 A1
- US 20030205229 A1
- US 20060201499 A1
- WO 9215353 A
- WO 9748431 A
- WO 2006013952 A1
- WO 0001434 A